# EUROPÄISCHE PATENTSCHRIFT

(11) **EP 0 538 189 B1**
(45) Veröffentlichungstag und Bekanntmachung des Hinweises auf die Patenterteilung: **10.01.1996**
(21) Anmeldenummer: 92810758.0
(22) Anmeldetag: 06.10.1992
(51) Int. Cl.: C07C 69/732, C07C 67/03

(54) **Verfahren zur Herstellung von Hydroxyphenylcarbonsäureestern**
Process for the preparation of esters of hydroxyphenyl carboxylic acids
Procédé de préparation d'esters d'acides hydroxyphénylcarboxyliques

(30) Priorität: 15.10.1991 CH 3028/91
(43) Veröffentlichungstag der Anmeldung: 21.04.1993
(73) Patentinhaber: CIBA-GEIGY AG, CH-4002 Basel (CH)
(72) Erfinder: Kleiner, Christoph, Dr., CH-5262 Frick (CH); Evans, Samuel, Dr., CH-1723 Marly (CH); Schmitt, Ralf, Dr., CH-4450 Sissach (DE)

(56) Entgegenhaltungen:
- EP-A- 0 148 729
- EP-A- 0 200 685
- FR-A- 1 490 341
- US-A- 3 944 594
- US-A- 4 112 240

## Beschreibung

Die Erfindung betrifft ein Verfahren zur Herstellung von Hydroxyphenylcarbonsäureestern sowie die Verwendung der eingesetzten Katalysatoren.

Die unten beschriebenen Hydroxyphenylcarbonsäureester der Formel I werden durch Umesterung nach einer Reihe bekannter Verfahren hergestellt (z.B. US-A-3,330,859; US-A-3,944,594; US-A-4,085,132; US-A-4,228,297; US-A-4,536,593; US-A-4,594,444; US-A-4,618,700; US-A-4,716,244). Diese Verfahren vermögen noch nicht voll zu befriedigen. So sind z.B. die als Katalysatoren eingesetzten Titanverbindungen häufig schwer von der Reaktionsmasse zu trennen; einmal verbraucht, müssen sie oft aufwendig zerstört, mit Hilfsstoffen filtriert und die Filtrationsrückstände einer Deponie zugeführt werden; insbesondere kann es durch Rückstände des Katalysators im Produkt zu unerwünschten Oxidationsreaktionen kommen, die eine Verfärbung der Produkte bewirken.

Es besteht daher weiterhin ein Bedarf an neuen, verbesserten Verfahren zur Herstellung dieser Verbindungen.

Aluminiumalkoholate sind bereits als Veresterungs- und Umesterungskatalysatoren bekannt. Sie wurden z.B. für die Herstellung von β-Phenylpropionsäureallylestern durch Umesterung eingesetzt (FR-A-1.490.341). Diese Verbindungen werden als Duftstoffe für die Parfümindustrie empfohlen.

Es wurde nun überraschenderweise gefunden, daß durch die Anwendung von Aluminiumalkoholaten als Katalysatoren die unten beschriebenen Hydroxyphenylcarbonsäureester sauber, in guten Ausbeuten, ohne Trenn- und Oxidationsprobleme und mit umweltgerechten Hilfsmitteln hergestellt werden können.

Die Erfindung betrifft somit ein Verfahren zur Herstellung von Verbindungen der Formel I 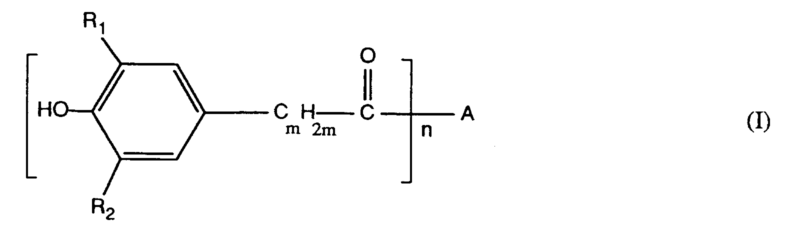
worin R₁ und R₂ unabhängig voneinander C₁-C₈-Alkyl sind,
m 0, 1, 2, oder 3 ist,
n 1 oder 2 bedeutet und
A für n = 1 OR₃ darstellt, wobei
R₃ C₄-C₂₀-Alkyl oder C₅-C₁₂-Cycloalkyl ist,
oder A für n = 2 den Formeln -O-CₓH₂ₓ-O- oder -O-(CH₂CH₂O)ₐCH₂CH₂O- entspricht,
x eine Zahl von 2 bis 8 und a eine Zahl von 1 bis 12 ist,
durch Umsetzung einer Verbindung der Formel II 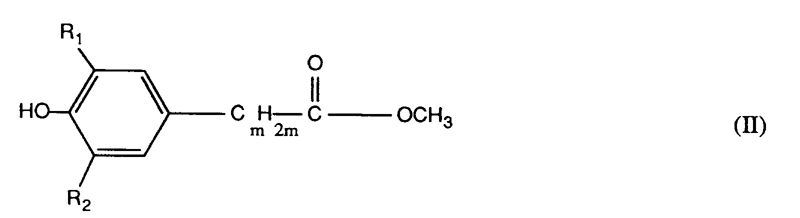
mit einer Verbindung der Formel III

A(̵H)ₙ , (III)

wobei die Umsetzung in Gegenwart eines Aluminiumtrialkoholates oder -phenolates als Katalysator durchgeführt wird.

Stellen R₁ und R₂ C₁-C₈-Alkyl dar, so handelt es sich dabei um verzweigte oder unverzweigte Reste. Beispiele hierfür sind Methyl, Ethyl, Propyl, Isopropyl, n-Butyl, Isobutyl, t-Butyl, Pentyl, Isopentyl, Hexyl, Heptyl, 3-Heptyl, Octyl, 2-Ethylbutyl, 1-Methylpentyl, 1,3-Dimethylbutyl, 1,1,3,3-Tetramethylbutyl, 1-Methylhexyl, Isoheptyl, 1-Methylheptyl und 2-Ethylhexyl. Für R₃ als C₄-C₂₀-Alkyl sind Beispiele der obigen Liste ab vier Kohlenstoffatomen zu entnehmen, zusätzlich kann R₃ noch z.B. Nonyl, Decyl, Undecyl, Dodecyl, Tridecyl, Tetradecyl, Pentadecyl, Hexadecyl, Heptadecyl, Octadecyl, Eicosyl, 1,1,3-Trimethylhexyl oder 1-Methylundecyl bedeuten.

Bevorzugt sind für R₁ und R₂ Alkylreste mit 1-4 C-Atomen. Beispiele dafür sind obiger Liste zu entnehmen.

R₃ als C₅-C₁₂-Cycloalkyl kann z.B. Cyclopentyl, Cyclohexyl, Cycloheptyl, Cyclooctyl oder Cyclododecyl sein. Cyclopentyl und Cyclohexyl sind bevorzugt, insbesondere Cyclohexyl.

Bevorzugt ist R₃ höheres Alkyl, z.B. C₈-C₂₀, besonders bevorzugt i-Octyl oder n-Octadecyl. Als Isooctyl wird 2-Ethylhexyl bezeichnet.

Vorzugsweise steht x für 4 bis 8 und a für 1 bis 4.

Das Verfahren wird bevorzugt zur Herstellung von Verbindungen der Formel I angewandt, worin m für 2 steht.

Besonders bevorzugt werden Verbindungen der Formel I hergestellt, worin R₁ und R₂ tert-Butyl darstellen, R₃ für n = 1 n-Octadecyl oder i-Octyl ist und A für n = 2 die Gruppe -O-(CH₂)₆-O- darstellt.

Ganz besonders bevorzugt werden Verbindungen der Formel I hergestellt, worin R₁ für Methyl, R₂ für tert-Butyl stehen, n = 2 ist und A der Formel -O-(CH₂CH₂O)₂CH₂CH₂O-entspricht.

Die erfindungsgemäße Verbesserung des Verfahrens besteht in der Anwendung von Aluminiumtrialkoholaten und -phenolaten als Katalysatoren. Die Erfindung betrifft daher auch die Verwendung von Aluminiumtrialkoholaten und -phenolaten als Katalysatoren bei der Herstellung von Verbindungen der Formel I durch Umsetzung von Verbindungen der Formel II mit Verbindungen der Formel III.

Als Katalysatoren kommen in Frage Verbindungen der Formel IV

Al(OR)₃, (IV)

wobei R ein aliphatischer oder aromatischer Rest sein kann.
zweckmäßige aliphatische Reste sind unsubstituiertes oder OH-substituiertes C₁-C₆-insbesondere C₁-C₄-Alkyl.
Als aromatischer Rest entspricht R der Formel 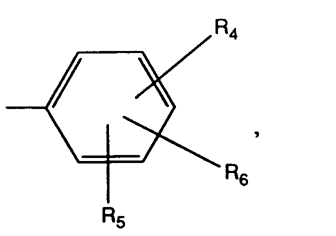
worin R₄ und R₅ unabhängig voneinander Wasserstoff oder C₁-C₄-Alkyl, besonders Methyl oder tert-Butyl, bedeuten und R₆ für Wasserstoff oder eine Gruppe der Formel 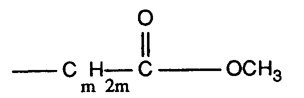
steht, wobei, wenn R₆ nicht Wasserstoff ist, dieses in 4-Stellung zum O-Atom angeordnet ist.

Als aromatischer Rest ist R bevorzugt ein solcher der Formel V 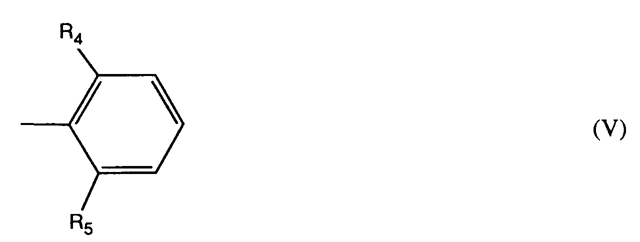
oder es entspricht z.B. der Formel VI, 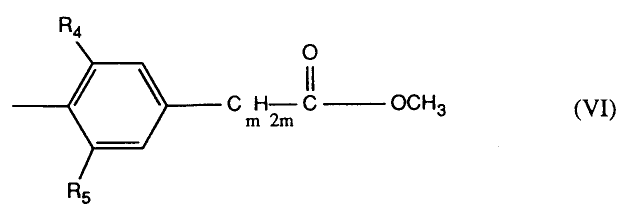
wobei in Formel VI R₄ und R₅ vorzugsweise von Wasserstoff verschieden sind.

Als aliphatischer Rest ist R bevorzugt Methyl, Ethyl, Isopropyl oder 2-Hydroxybutyl, besonders bevorzugt Isopropyl.

Besonders vorteilhaft ist die Verwendung einer Mischung von 65% Aluminiumtriisopropylat, 30% Siedegrenzbenzin, 4% Isooctanol und 1% Isopropanol als Katalysatorzubereitung.

Das erfindungsgemäße Verfahren kann in einem inerten organischen Lösungsmittel durchgeführt werden, beispielsweise in aliphatischen oder aromatischen Kohlenwasserstoffen wie Pentan, Hexan, Heptan, Octan, Cyclohexan, Decalin, Petrolether oder Mischungen davon bzw. Benzol, Toluol oder Xylol(en);.

Die Reaktionspartner der Formeln II und III werden zweckmäßig zur homogenen Schmelze gebracht, bevor der Katalysator zugegeben wird Bevorzugt werden sie unter vermindertem Druck (beispielsweise zwischen 2 und 200 mbar, vorteilhaft bei 20 mbar) erhitzt, bis eine Schmelze vorliegt. Dies dient auch der Vortrocknung der Reaktionspartner. Empfehlenswerte Temperatur dafür ist z.B. 80-90°C.

Der Katalysator wird dem Reaktionsgemisch zweckmäßig in Mengen von 0.05 bis 10 mol%, bevorzugt von 0.05 bis 5 mol%, besonders bevorzugt 0.1 bis 2 mol%, bezogen auf die Verbindungen der Formel II, zugegeben.

Übliche Operationen wie Rühren des Reaktionsgemisches sind von Vorteil.

Die Reaktionstemperatur liegt zweckmäßig bei 120 bis 200°C, bevorzugt bei 140 bis 180°C, besonders bevorzugt bei 150 bis 170°C.

Die Reaktionszeit kann in weiten Grenzen schwanken und liegt im allgemeinen je nach Druck und Temperatur zwischen 2 und 12 Stunden.

Der Druck während der Reaktionszeit beträgt zweckmäßig 1 bis 200 mbar, z.B. 1 bis 50, bevorzugt 1 bis 15 mbar. Da während der Reaktion Methanol frei wird, kann der Druck sich im Verlauf der Reaktion ändern. Z.B. steigt er in dem Maße, wie Methanol freigesetzt wird. Ist dieses abgetrennt, so verringert man zweckmäßig den Druck weiter, bis die gegebenenfalls überschüssige Komponente III abgetrennt ist.

Nach beendeter Reaktion wird eventuell aus wäßrigen Verunreinigungen entstandenes Aluminiumhydroxid zweckmäßig abfiltriert.

Zur Entfernung des Katalysators wird dieser im allgemeinen mit einer geeigneten Säure versetzt. Dafür kommen z. B. Essig- und Ameisensäure oder eine Mischung aus beiden in Frage. In einer bevorzugten Ausführungsform arbeitet man mit Essigsäure, und zwar insbesondere mit einem mindestens 3-fachen molaren Überschuß Essigsäure, bezogen auf die Katalysatormenge, um diesen zu Aluminiumacetat umzusetzen. Bevorzugt ist ein 3 bis 6-facher Überschuß, insbesondere ein fünffacher. Dazu ist es zweckmäßig, die Reaktionsmischung 30 Minuten bis 2 Stunden bei 80 bis 110°C mit der Essigsäure zu rühren. Erfolgt die weitere Aufarbeitung mit einem Lösungsmittel, so bleibt der überwiegende Teil des Aluminiumacetats in Lösung, während das Produkt kristallisiert werden kann.

In einer weiteren, bevorzugten Ausführungsform wird der Katalysator mit Ameisensäure zerstört, die vorzugsweise in mindestens dreifachem, bis zu 20 fachem Überschuß (bezogen auf Al(ⁱPr)₃eingesetzt wird. Bewährt hat sich beispielsweise ein zehnfacher Überschuß. Es ist vorteilhaft, das Reaktionsgemisch 1/2 bis 2 Stunden bei 80 - 100°C, bevorzugt bei 90°C mit Ameisensäure zu rühren. Beim Stehenlassen trennen sich die zwei Phasen des Gemischs. Die untere, wäßrige Phase enthält die Ameisensäure und das Aluminiumsalz und ist weitgehend homogen, so daß eine Trennung von der organischen, das Produkt enthaltenden Phase ohne Schwierigkeiten möglich ist.

Bei Kristallisation direkt aus der Schmelze weist das Endprodukt einen erhöhten Aluminiumanteil auf, der bei der Verwendung als Stabilisator für gewöhnlich nicht stört.

Das Produkt der Formel I kann also entweder direkt durch Abkühlen und gegebenenfalls Impfen der Reaktionsschmelze zur Kristallisation gebracht werden, oder die Reaktionsschmelze wird in einem geeigneten Lösungsmittel aufgenommen, abgekühlt und gegebenenfalls unter Impfen kristallisiert. Als Lösungsmittel kommen in Frage: aliphatische Kohlenwasserstoffe wie Pentan, Hexan, Heptan, Octan, Cyclohexan, Decalin, Petrolether oder Mischungen davon; aromatische Kohlenwasserstoffe wie Benzol, Toluol oder Xylol; Alkohole und Alkohol/Wasser-Gemische wie Ethanol (80-100%), Methanol (80-100%) und Isopropanol (80-100%). Bevorzugt sind Alkohol-Wasser Gemische, insbesondere Methanol (80-100%).

In der Regel setzt man ungefähr äquivalente Mengen des Esters II und des Alkohols III ein. Zweckmäßig liegt das Verhältnis Reaktand II pro Äquivalent des Reaktanden III zwischen und 0.8 : 1 und 1.3 : 1, vorteilhaft: zwischen 0.85 : 1 und 1.2 : 1.

Besonders hervorzuheben ist die Tatsache, daß bei dem erfindungsgemäßen Verfahren Verfärbungen in der Reaktionsmasse und in den Produkten vermieden werden. Die eingangs erwähnten Verfärbungsprobleme infolge Oxidation durch Katalysatorrückstände unterbleiben.

Das Verfahren zeichnet sich ferner dadurch aus, daß ein Filtrationsschritt nicht unbedingt vonnöten ist, und daß die Anzahl der Nebenkomponenten erfreulich gering ist. Allfällige Katalysatorreste stören im Endprodukt für die beabsichtigte Verwendung als Stabilisator nicht. Wird aus Methanol kristallisiert, so verbleiben gewöhnlich weniger als 10 ppm Aluminium im Endprodukt.

Die im erfindungsgemäßen Verfahren eingesetzten Verbindungen der Formeln II , III und IV sind bekannt oder können nach an sich bekannten Verfahren hergestellt werden. In den eingangs zitierten Dokumenten sind Verbindungen der Formeln II und III beschrieben.

Die erfindungsgemäß hergestellten Verbindungen der Formel I dienen beispielsweise dem Schutz von thermischem, oxidativem und/oder actinischem Abbau unterliegenden organischen Materialien wie z.B. Kunststoffen und Schmierstoffen und sind zum Teil im Handel erhältlich.

Die folgenden Beispiele erläutern die Erfindung weiter, ohne sie jedoch zu beschränken. Alle Angaben in Prozenten und Teilen beziehen sich auf das Gewicht, sofern nicht anders angegeben.

### Beispiel 1:

### Bis[β-(3-tert-Butyl-5-methyl-4-hydroxyphenyl)propionsäure]triethylenglykolester (Verbindung der Formel I, worin R₁ tert-Butyl und R₂ Methyl sind, n und m jeweils 2 sind und A der Formel -O-(CH₂CH₂O)₂CH₂CH₂O- entspricht)

In einem 1 l Sulfierkolben werden 266 g
β-(3-tert-Butyl-5-methyl-4-hydroxyphenyl)propionsäuremethylester und 78 g Triethylenglykol vorgelegt. Die Apparatur wird verschlossen, evakuiert und mit Stickstoff entlastet. Anschließend wird bei 90°C und 20 mbar eine Stunde lang getrocknet. Danach werden 1.46 g Aluminumtriisopropylat zugegeben und auf 3 mbar evakuiert. Dann wird während ca. einer Stunde auf 160°C aufgeheizt, wobei über den mit 60°warmem Wasser beheizten Rückflußkühler ab ca 135° das Methanol abgetrieben wird. Dieses Reaktionsmethanol wird in einer Kühlfalle kondensiert: Es werden nach einer Haltezeit von 8 Stunden ca. 36 g erhalten. Die Apparatur wird anschließend mit Stickstoff entlastet und die Reaktionsmasse während 30 Minuten mit 6 ml Essigsäure bei 100°C verrührt. Dann wird filtriert und aus 360 ml 80%igem Methanol kristallisiert. Ausbeute 282 g (90%), Smp. 76-79°C, weißes Pulver.

### Beispiel 2: β-(3,5-Di-tert-butyl-4-hydroxyphenyl)propionsäurestearylester (Verbindung der Formel I, worin R₁ und R₂ tert-Butyl sind, n = 1 und m = 2 sind, und A für -O-ⁿC₁₈H₃₇steht)

202 g β-(3,5-Di-tert-butyl-4-hydroxyphenyl)propionsäuremethylester und 185 g Stearylalkohol (trocken) werden vorgelegt und bei 80°C und 200 mbar geschmolzen. Wenn alles geschmolzen ist, wird das Vakuum mit Stickstoff gebrochen und es werden 1.4 g Aluminiumtriisopropylat zugegeben. Es wird auf 3 mbar evakuiert und binnen einer Stunde auf 170°C aufgeheizt. Bei dieser Temperatur wird 3 Stunden gerührt. Die mit Essigsäure angesäuerte Reaktionsschmelze wird zur Kristallisation stehen gelassen oder in Methanol(97%) aufgenommen und kristallisiert. Ausbeute 95.5%; Smp. 53°C.

### Beispiel 3: Bis[β-(3,5-di-tert-butyl-4-hydroxyphenyl)propionsäure]hexandiolester (Verbindung der Formel I, worin R₁ und R₂ tert-Butyl sind, n und m = 2 sind und A die Gruppe -O-(CH₂)₆-O- darstellt)

320 g β-(3,5-Di-tert-butyl-4-hydroxyphenyl)propionsäuremethylester und 64 g Hexandiol (trocken) werden vorgelegt und bei 80°C und 200 mbar geschmolzen. Wenn alles geschmolzen ist, wird das Vakuum mit Stickstoff gebrochen und es werden 2.2 g Aluminiumtrisopropylat zugegeben. Es wird auf 3 mbar evakuiert und binnen einer Stunde auf 150°C aufgeheizt. Bei dieser Temperatur wird 5 Stunden gerührt. Die mit Essigsäure angesäuerte Reaktionsschmelze wird in Methanol(97%) aufgenommen, mit 5% Wasser versetzt und kristallisiert. Ausbeute 90%; Smp. 103-108°C.

### Beispiel 4: β-(3,5-Di-tert-butyl-4-hydroxyphenyl)propionsäureisooctylester (Verbindung der Formel I, worin R₁ und R₂ tert-Butyl sind, n = 1 und m = 2 sind, und A für -O-ⁱC₈H₁₇ steht)

393 g β-(3,5-Di-tert-butyl-4-hydroxyphenyl)propionsäuremethylester und 201.1 g wasserfreies (H₂O < 0.1 Gew.-%) Isooctanol werden vorgelegt. Anschließend wird die Reaktionsmasse geschmolzen, wobei die Temperatur 70°C erreicht. Nun werden 4.5 g Aluminiumtriisopropylat als Feststoff zugegeben. Die Apparatur wird verschlossen, evakuiert und mit Stickstoff entlastet. Nach der Katalysatordosierung wird auf die Reaktionstemperatur von 150 bis 160°C aufgeheizt. Das entstehende Methanol wird bei zunehmend gesteigertem Vakuum (bis 20 mbar) vollständig abdestilliert. In der Destillationsvorlage werden insgesamt 43.1 g Methanol abgetrennt. Nach einer Reaktionszeit von ca. 5 Stunden wird das überschüssige Isooctanol bei einem allmählich gesteigerten Vakuum von 5 bis 1 mbar fast vollständig abdestilliert. Es kann ohne Qualitätseinbußen rezykliert werden. Die verbleibende Reaktionsmasse wird auf eine Innentemperatur von 90°C abgekühlt und mit 171.8 g Ameisensäure (6%ig) versetzt, eine halbe Stunde lang bei 90°C gerührt und zur Phasentrennung eine weitere halbe Stunde stehen gelassen. Die Ameisensäure und Aluminiumsalze enthaltende untere, wäßrige Phase ist weitgehend homogen und wird von der organischen, das Produkt enthaltenden Phase getrennt. Aluminiumsalze bleiben durch die vorgelegte Ameisensäure in der wäßrigen Phase gelöst. Anschließend wird die organische Phase bei 90°C zweimal mit jeweils 170 g Wasser nachgewaschen, trockendestilliert und über einen thermostatisierten Linsenfilter filtriert.

Ausbeute 99.5%; n_{D}²⁰ 1.499.

## Patentansprüche

1. Verfahren zur Herstellung von Verbindungen der Formel I 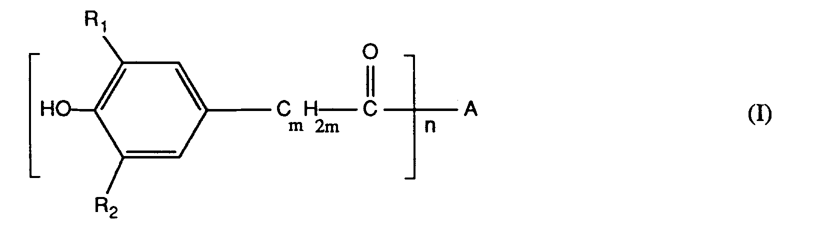 worin R₁ und R₂ unabhängig voneinander C₁-C₈-Alkyl sind,
m 0, 1, 2, oder 3 ist,
n 1 oder 2 bedeutet und
A für n = 1 OR₃ darstellt, wobei
R₃ C₄-C₂₀-Alkyl oder C₅-C₁₂-Cycloalkyl ist,
oder A für n = 2 den Formeln -O-CₓH₂ₓ-O- oder -O-(CH₂CH₂O)ₐCH₂CH₂O- entspricht,
x eine Zähl von 2 bis 8 und a eine Zähl von 1 bis 12 ist,
durch Umsetzung einer Verbindung der Formel II 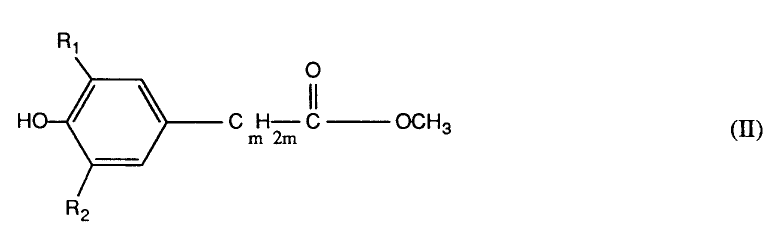 mit einer Verbindung der Formel III
A(̵H)ₙ , (III)
dadurch gekennzeichnet, daß die Umsetzung in Gegenwart eines Aluminiumtrialkoholates oder -phenolates als Katalysator durchgeführt wird.

2. Verfahren nach Anspruch 1, dadurch gekennzeichnet, daß m für 2 steht.

3. Verfahren nach Anspruch 1, dadurch gekennzeichnet, daß R₁ und R₂ C₁-C₄-Alkyl sind.

4. Verfahren nach Anspruch 1, dadurch gekennzeichnet, daß R₃ C₈-C₂₀-Alkyl ist, x 4 bis 8 ist und a 1 bis 4 bedeutet.

5. Verfahren nach Anspruch 2, dadurch gekennzeichnet, daß R₁ und R₂ tert-Butyl darstellen, R₃ für n = 1 n-Octadecyl oder i-Octyl ist und A für n = 2 die Gruppe -O-(CH₂)₆-O- darstellt.

6. Verfahren nach Anspruch 2, dadurch gekennzeichnet, daß R₁ für Methyl und R₂ für tert-Butyl stehen, n = 2 ist und A der Formel -O-(CH₂CH₂O)₂CH₂CH₂O- entspricht.

7. Verfahren nach Anspruch 1, dadurch gekennzeichnet, daß ein Katalysator der Formel Al(OR)₃ eingesetzt wird, worin R die Bedeutung von unsubstituiertem oder OH-substituiertem C₁-C₆-Alkyl hat oder ein Rest der Formel 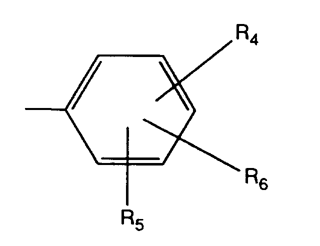 ist, worin R₄ und R₅ unabhängig voneinander Wasserstoff oder C₁-C₄-Alkyl bedeuten und R₆ für Wasserstoff oder eine Gruppe der Formel 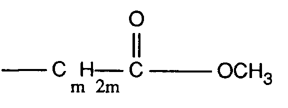 steht, wobei, wenn R₆ nicht Wasserstoff ist, dieses in 4-Stellung zum O-Atom angeordnet ist.

8. Verfahren nach Anspruch 7, dadurch gekennzeichnet, daß R unsubstituiertes oder OH-substituiertes C₁-C₆-Alkyl bedeutet.

9. Verfahren nach Anspruch 1, dadurch gekennzeichnet, daß Aluminiumtriisopropylat als Katalysator eingesetzt wird.

10. Verfahren nach Anspruch 1, dadurch gekennzeichnet, daß die Umsetzung bei Temperaturen von 120 bis 200°C durchgeführt wird.

11. Verfahren nach Anspruch 1, dadurch gekennzeichnet, daß der Druck während der Umsetzung 1 bis 200 mbar beträgt.

12. Verwendung von Aluminiumtrialkoholaten und -phenolaten als Katalysatoren bei der Herstellung von Verbindungen der Formel I gemäß Anspruch 1 durch Umsetzung von Verbindungen der Formel II gemäß Anspruch 1 mit Verbindungen der Formel III gemäß Anspruch 1.

13. Verfahren nach Anspruch 1, dadurch gekennzeichnet daß nach der Reaktion der Katalysator durch Zugabe einer Säure, insbesondere Essig-oder Ameisensäure, zerstört wird.

## Claims

1. A process for the preparation of a compound of formula I 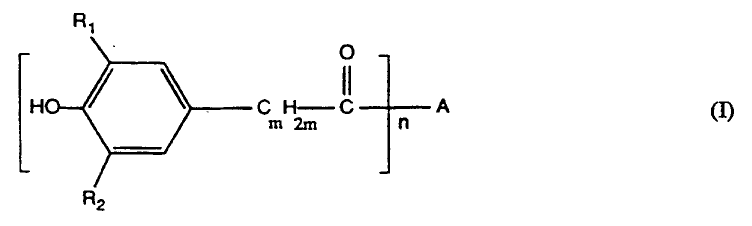 in which R₁ and R₂ are each independently of the other C₁-C₈alkyl,
m is 0, 1, 2 or 3,
n is 1 or 2, and
A, if n = 1, is OR₃, and
R₃ is C₄-C₂₀alkyl or C₅-C₁₂cycloalkyl,
or A, if n = 2, has the formula -O-CₓH₂ₓ-O- or -O-(CH₂CH₂O)ₐCH₂CH₂O-,
x is a number from 2 to 8 and a is a number from 1 to 12,
by reacting a compound of formula II 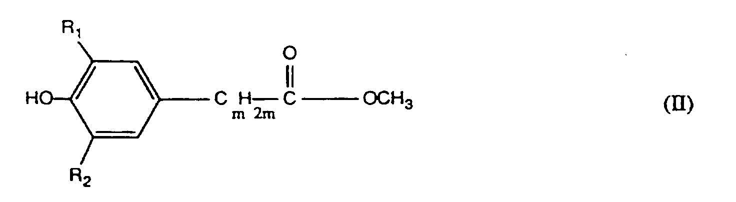 with a compound of formula III
A(̵H)ₙ , (III)
wherein the reaction is carried out in the presence of an aluminium trialcoholate or triphenolate as catalyst.

2. A process according to claim 1, wherein m is 2.

3. A process according to claim 1, wherein R₁ and R₂ are C₁-C₄alkyl.

4. A process according to claim 1, wherein R₃ is C₈-C₂₀alkyl, x is from 4 to 8 and a is from 1 to 4.

5. A process according to claim 2, wherein R₁ and R₂ are tert-butyl, R₃, if n = 1, is n-octadecyl or isooctyl, and A, if n = 2, is the group -O-(CH₂)₆-O-.

6. A process according to claim 2, wherein R₁ is methyl and R₂ is tert-butyl, n = 2 and A has the formula -O-(CH₂CH₂O)₂CH₂CH₂O-.

7. A process according to claim 1, which comprises the use of a catalyst of formula Al(OR)₃ in which R is unsubstituted or OH-substituted C₁-C₆alkyl or is a radical of formula 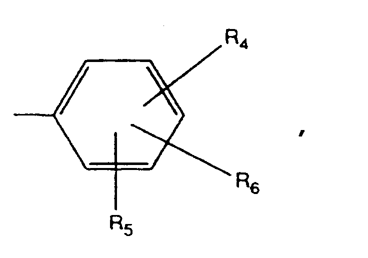 in which R₄ and R₅ are each independently of the other hydrogen or C₁-C₄alkyl, and R₆ is hydrogen or a group of formula 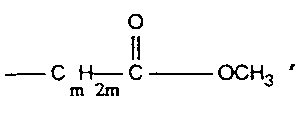 with the proviso that R₆, if different from hydrogen, is in 4-position to the oxygen atom.

8. A process according to claim 7, wherein R is unsubstituted or OH-substituted C₁-C₆alkyl.

9. A process according to claim 1, wherein the catalyst employed is aluminium triisopropylate.

10. A process according to claim 1, wherein the reaction is carried out at temperatures from 120 to 200°C.

11. A process according to claim 1, wherein the pressure during the reaction is from 1 to 200 mbar.

12. Use of an aluminium trialcoholate or triphenolate as catalyst in the preparation of a compound of formula I according to claim 1, by reacting a compound of formula II according to claim 1 with a compound of formula III according to claim 1.

13. A process according to claim 1, wherein after the reaction the catalyst is destroyed by addition of an acid, especially acetic or formic acid.

## Revendications

1. Procédé pour préparer des composés de formule (I) : 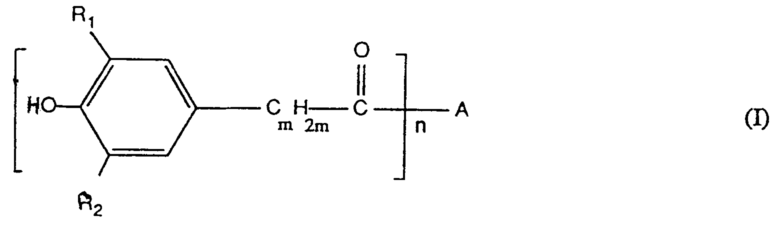 dans laquelle R₁ et R₂, indépendamment l'un de l'autre, sont des radicaux alkyle en C₁-C₈,
m vaut 0, 1, 2 ou 3,
n vaut 1 ou 2, et
A, quand n = 1, représente OR₃, où R₃ est un radical alkyle en C₄-C₂₀ ou cycloalkyle en C₅-C₁₂,
ou bien A, quand n vaut 2, correspond aux formules -O-CₓH₂ₓ-O- ou O-(CH₂CH₂O)ₐCH₂CH₂O-,
x est un nombre de 1 à 8 et a est un nombre de 1 à 12,
par la réaction d'un composé de formule (II) : 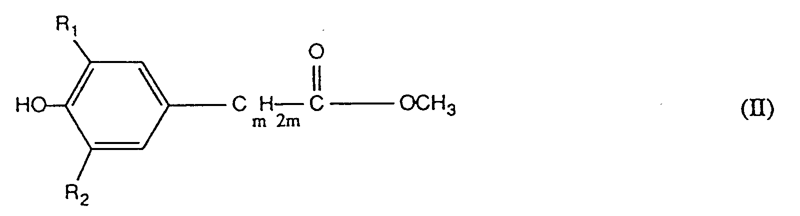 avec un composé de formule (III) :
A(̵H)ₙ (III)
caractérisé en ce que la réaction est mise en oeuvre en présence d'un trialcoolate ou phénolate d'aluminium servant de catalyseur.

2. Procédé selon la revendication 1, caractérisé en ce que m vaut 2.

3. Procédé selon la revendication 1, caractérisé en ce que R₁ et R₂ sont des radicaux alkyle en C₁-C₄.

4. Procédé selon la revendication 1, caractérisé en ce que R₃ est un radical alkyle en C₈-C₂₀, x vaut de 4 à 7, et a vaut de 1 à 4.

5. Procédé selon la revendication 2, caractérisé en ce que R₁ et R₂ sont des radicaux tert-butyle, R₃, quand n vaut 1, est le radical n-octadécyle ou isooctyle, et A, quand n vaut 2, est le groupe -O-(CH₂)₆-O-.

6. Procédé selon la revendication 2, caractérisé en ce que R₁ est le radical méthyle et R₂ le radical tert-butyle, n vaut 2 et A correspond à la formule -O-(CH₂CH₂O)₂CH₂CH₂O-.

7. Procédé selon la revendication 1, caractérisé en ce qu'on utilise un catalyseur de formule Al(OR)₃, dans laquelle R représente un radical alkyle en C₁-C₆ non substitué ou hydroxylé, ou encore est un radical de formule : 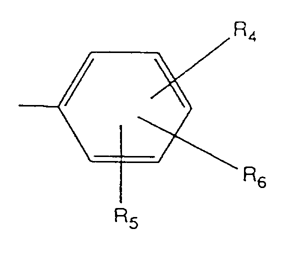 dans laquelle R₄ et R₅, indépendamment l'un de l'autre, sont des hydrogènes ou des radicaux alkyle en C₁-C₄, et R₆ est un hydrogène ou un groupe de formule : 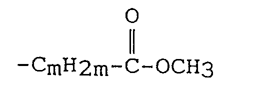 où, quand R₆ n'est pas un hydrogène, ce radical est disposé en position 4 par rapport à l'atome d'oxygène.

8. Procédé selon la revendication 7, caractérisé en ce que R est un radical alkyle en C₁-C₆ non substitué ou hydroxylé.

9. Procédé selon la revendication 1, caractérisé en ce que le triisopropylate d'aluminium est utilisé comme catalyseur.

10. Procédé selon la revendication 1, caractérisé en ce que la réaction est mise en oeuvre à des températures de 120 à 200°C.

11. Procédé selon la revendication 1, caractérisé en ce que la pression, pendant la réaction, est de 1 à 200 mbar.

12. Utilisation de trialcoolates d'aluminium et phénolates d'aluminium comme catalyseurs lors de la préparation de composés de formule (I) selon la revendication 1, par réaction de composés de formule (II) selon la revendication 1 avec des composés de formule (III) selon la revendication 1.

13. Procédé selon la revendication 1, caractérisé en ce que, après la réaction, le catalyseur est décomposé par addition d'un acide, en particulier l'acide acétique ou l'acide formique.
